(19) Europäisches Patentamt
European Patent Office
Office européen des brevets

(11) Veröffentlichungsnummer: **0 023 661**
**B1**

(12) # EUROPÄISCHE PATENTSCHRIFT

(45) Veröffentlichungstag der Patentschrift: **04.08.82**

(51) Int Cl.³: **G 03 C 1/34**

(21) Anmeldenummer: **80104282.1**

(22) Anmeldetag: **21.07.80**

(54) **Photographische Silberhalogenidemulsion mit einem Stabilisator, Verfahren zu ihrer Herstellung, photographische Materialien sowie Verfahren zur Herstellung photographischer Bilder.**

(30) Priorität: **02.08.79 DE 2931468**

(43) Veröffentlichungstag der Anmeldung: **11.02.81 Patentblatt 81/6**

(45) Bekanntmachung des Hinweises auf die Patenterteilung: **04.08.82 Patentblatt 82/31**

(84) Benannte Vertragsstaaten: **BE DE FR GB**

(56) Entgegenhaltungen:
**DE-A-2 045 724**
**DE-A-2 508 137**
**US-A-3 488 709**
**US-A-3 737 313**
**RESEARCH DISCLOSURE August 1976, Nr. 148, Industrial Opportunities Ltd, Havant, Hampshire, GB, »Use of polyazaindenes in photographic emulsions«, Seiten 51—53 »Disclosure«, Nr. 14 851.**

(73) Patentinhaber: **AGFA-GEVAERT Aktiengesellschaft, D-5090 Leverkusen 1 (DE)**

(72) Erfinder: **De Brabandere, Luc A. Dr., Zevenbergen 52, B-2500 Lier (BE)**
Erfinder: **Gernert, Herbert, Dr., Solothurner Strasse 28, D-8000 München 55 (DE)**
Erfinder: **von König, Anita, Dr., Schönwasserstrasse 230, D-4150 Krefeld 1 (DE)**
Erfinder: **Philippaerts, Herman A. Dr., 11, Mgr. J. Vandeveldelaan, B-2520 Edegem (BE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 661 B1

BUNDESDRUCKEREI BERLIN

# Photographische Silberhalogenidemulsion mit einem Stabilisator, Verfahren zu ihrer Herstellung, photographische Materialien sowie Verfahren zur Herstellung photographischer Bilder

Die Erfindung betrifft eine Silberhalogenidemulsion, die durch den Zusatz wenigstens eines Stabilisierungsmittels gegen die Bildung von Schleier und gegen eine Verflachung der Gradation stabilisiert ist. Weiterhin betrifft die Erfindung ein Verfahren zur Herstellung einer derartigen Emulsion, photographische Materialien sowie ein Verfahren zur Herstellung photographischer Bilder.

Materialien mit lichtempfindlichen Silberhalogenidemulsionen, insbesondere chemisch sensibilisierte, neigen bekanntlich zur Bildung von Schleiern, hervorgerufen durch Keime, die ohne Belichtung entwickelbar sind. Diese Schleierbildung tritt insbesondere auf bei zu langer Lagerung, besonders bei erhöhter Temperatur und Luftfeuchtigkeit, bei zu langer Entwicklung oder Entwicklung bei zu hohen Temperaturen durch bestimmte Zusätze und bei stark gereiften Emulsionen.

Es ist bekannt, photographischen Silberhalogenidemulsionen zur Verminderung dieser Schleierbildung sogenannte Antischleiermittel oder Stabilisierungsmittel zuzusetzen. Stabilisierende Wirkung besitzen z. B. heterocyclische Mercaptoverbindungen, z. B. solche, die in DE-B-1 183 371, DE-A-2 308 530 und DE-A-1 622 271 beschrieben sind.

Diesen Stabilisierungsmitteln haftet jedoch als Nachteil an, daß sie in wirksamen Konzentrationen im allgemeinen die Empfindlichkeit der stabilisierten Emulsion herabsetzen, wodurch deren Anwendbarkeit beeinträchtigt wird. Auch die Gradation der Emulsion kann durch diese Stabilisatoren ungünstig beeinflußt werden.

Bekannte Stabilisierungsmittel für lichtempfindliche photographische Silberhalogenidemulsionen sind weiterhin bestimmte Azainden-Derivate, die im allgemeinen den Schleier einer photographischen Silberhalogenidemulsion unterdrücken ohne in störendem Umfange die Empfindlichkeit der Emulsion herabzusetzen.

An Stabilisierungsmittel werden aber in zunehmendem Maße, insbesondere auch in bezug auf die Wechselwirkung mit anderen photographischen Zusätzen und im Hinblick auf die Vielfältigkeit photographischer Reproduktionsprozesse und der hierfür verwendeten photographischen Materialien, steigende Anforderungen gestellt, denen die bekannten Stabilisierungsmittel nicht mehr ohne weiteres genügen.

Schließlich ist es bekannt, daß bei photographischen Materialien, insbesondere bei Materialien mit relativ steiler Gradation, bei Lagerung eine Verflachung der Gradation auftreten kann. Aus der US-A-3 488 709 ist es bekannt, die Gradation von Emulsionen, die Rhodiumsalze zur Gradationsaufsteilung enthalten, durch Zugabe von Cadmiumsalzen zu stabilisieren.

Weitere Verfahren zur Gradationsstabilisierung sind bekannt aus den deutschen Offenlegungsschriften 2 632 202 und 2 431 225. Die bekannten Verfahren genügen aber nicht mehr den heutigen Anforderungen an Stabilisierungsmittel.

Es besteht also weiterhin ein Bedarf an Stabilisierungsmitteln, die photographische Emulsionen bzw. Materialien gegen Schleier und Gradationsverflachung stabilisieren.

Der Erfindung lag die Aufgabe zugrunde, Stabilisierungsmittel aufzufinden, die gegen Schleier und Gradationsverflachung, insbesondere in den Spitzlichtern, stabilisieren. Es wurde nun ein photographisches Material mit wenigstens einer Silberhalogenidemulsionsschicht und wenigstens einem Stabilisierungsmittel gemäß folgender Formel I gefunden:

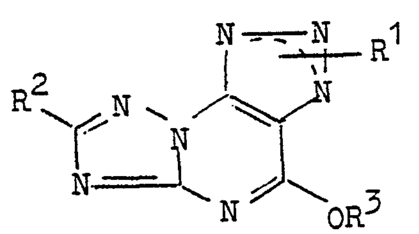

I

worin bedeuten:

$R^1$ H; Alkyl, vorzugsweise mit 1—4 C-Atomen; Cycloalkyl, vorzugsweise mit maximal 6 C-Atomen; Aryl, vorzugsweise Phenyl; Aralkyl, insbesondere Benzyl; Carbamoyl; Alkoxycarbonyl, vorzugsweise mit einem Alkylrest mit maximal 6 C-Atomen;

$R^2$ H; Alkyl, vorzugsweise mit maximal 6 C-Atomen; Cycloalkyl, vorzugsweise mit maximal 6 C-Atomen; Aryl, vorzugsweise Phenyl; Aralkyl, insbesondere Benzyl;

$R^3$ ein Kation, insbesondere $NH_4^+$, Wasserstoff oder ein Metallkation, vorzugsweise Natrium oder Kalium.

In einer besonders bevorzugten Ausführungsform bedeutet $R^1$: Wasserstoff, Carbamoyl oder Alkoxycarbonyl mit maximal 6 C-Atomen.

Die Carbamoylgruppe kann substituiert oder unsubstituiert sein. Substituierte Carbamoylgruppen

sind vorzugsweise Alkyl-, Aryl- oder Cycloalkylcarbamoylgruppen. In einer bevorzugten Ausführungsform bedeuten

$R^1$: Wasserstoff, Alkoxycarbonyl oder Carbamoyl
$R^2$: Wasserstoff, Methyl oder Phenyl
$R^3$: Wasserstoff, Natrium, Kalium oder Ammonium

Die erfindungsgemäß zu verwendenden Verbindungen können in folgenden tautomeren bzw. isomeren Formen II und III vorliegen:

II

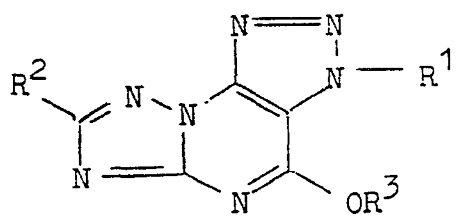

III

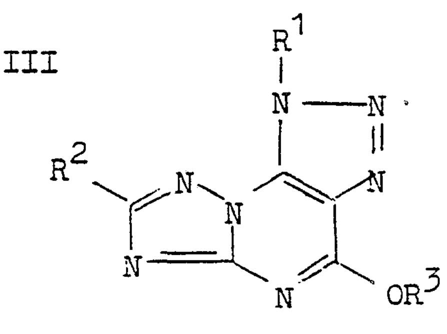

Die genannten Alkyl-, Cycloalkyl- und Arylreste können weitere übliche Substituenten enthalten, beispielsweise Alkyl oder Alkoxy, insbesondere mit maximal 4 C-Atomen; Halogen, insbesondere Cl.

Beispiele für erfindungsgemäß verwendbare Verbindungen der Formel I sind in der folgenden Tabelle I aufgeführt:

Tabelle I

| Verb. Nr. | $R^1$ | $R^2$ | $R^3$ | Schmelzpunkt (°C) |
|---|---|---|---|---|
| 1.1 | H | H | Na × 3 $H_2O$ | >360 |
| 1.2 | H | $CH_3$ | Na × 2 $H_2O$ | >360 |
| 1.3 | H | $C_6H_5$ | H × 1/2 $H_2O$ | 325–326 |
| 1.4 | $COOC_2H_5$ | H | H | 195–196 |

Die Verbindungen gemäß Formel I können nach prinzipiell bekannten Methoden hergestellt werden. Dazu wird aus dementsprechend substituierten 3-Amino-1,2,4-triazol und Cyanessigester das 5-Amino-7-hydroxy-1,3,8-triazaindolizin hergestellt.

Durch Kupplung mit diazotierter Sulfanilsäure und Reduktion des entstandenen Azofarbstoffs wird daraus das 5,6-Diamino-7-hydroxy-1,3,8-triazaindolizin erhalten. Durch Ringschluß mit Natriumnitrit wird daraus das gewünschte Indolizinderivat mit einem ankondensierten Triazolring hergestellt.

Beispielhaft wird im folgenden die Darstellung der Verbindung 1.1 beschrieben:

## Herstellung der Verbindung 1.1

Eine Suspension von 168 g 1H-1,2,4-triazol-3-amin, 360 ml 30%iger Natriummethylatlösung und 220 ml Cyanessigester in absolutem Alkohol wird 3 Stunden unter Rühren am Rückfluß erhitzt. Nach Abkühlung wird das ausgefallene Produkt abgesaugt und zweimal mit Alkohol verrührt und durch Umfällen aus Natronlauge mit Eisessig gereinigt.

Ausbeute: 174 g (≙55% der Theorie) 5-Amino-7-hydroxy-1,3,8-triazaindolizin.

51,9 g 90%iger Sulfanilsäure werden in 150 ml 10%iger Natronlauge gelöst und unter Kühlung mit Eis in 300 ml 10%iger Salzsäure ausgefällt. Die Suspension wird bei 2—4°C mit einer Lösung von 21 g Natriumnitrit in 60 ml Wasser diazotiert. Nach zweistündigem Nachrühren wird die Diazoniumsalzsuspension in 30 Minuten zu einer Lösung von 45,3 g 5-Amino-7-hydroxy-1,3,8-triazaindolizin in 375 ml 10%iger Natronlauge bei 8—10°C zugesetzt. Nach zweistündigem Nachrühren ohne Kühlung wird der ausgefallene Farbstoff abgesaugt und in 1900 ml Wasser gelöst, dann im Autoklaven bei 80 atm. und 85°C mit Raney-Nickel hydriert. Nach Absaugen des Katalysators wird im Filtrat durch Ansäuern mit

Eisessig das Diamin ausgefällt. Das abgesaugte Produkt wird nach Waschen mit Wasser im Vakuum bei 60° C getrocknet.

Ausbeute: 34,3 g (68,9% der Theorie) 5,6-Diamino-7-hydroxy-1,3,8-triazaindolizin.

66,4 g 5,6-Diamino-7-hydroxy-1,3,8-triazaindolizin werden in 55,3 g Kaliumcarbonat gelöst in 1600 ml Wasser durch Erwärmen gelöst. Nach Abkühlung auf 5—10° C wird mit Salzsäure auf pH 4 angesäuert und 90 ml 30%ige Natriumnitritlösung langsam unter Rühren bei 5—10°C zugetropft. Nach 1,5stündigem Nachrühren ohne Kühlung wird durch Ansäuern mit Salzsäure das Produkt ausgefällt. Das abgesaugte Produkt wird durch Waschen mit Wasser, Umfällen aus Sodalösung mit Salzsäure und Ansäuern bis ~ pH 5,5 und anschließendes Umkristallisieren aus Wasser gereinigt.

Ausbeute: 63 g (≙77% der Theorie) der Verbindung 1.1.

| | %, berechnet (mit 3 $H_2O$) | %, gefunden |
|---|---|---|
| C | 23,72 | 23,4/23,5 |
| H | 3,19 | 3,1/ 3,2 |
| N | 38,73 | 39,0/39,2 |
| O | 25,28 | 25,3/25,4 |
| Na | 9,08 | 9,4 |

Herstellung der Verbindung 1.4

Verbindung 1.1 wird in verdünnter Natronlauge gelöst und durch Ansäuern bis pH 1 ausgefällt. Nach Waschen mit Wasser und Trocknen werden 18,5 g dieser Verbindung mit 55 ml Pyrokohlensäurediethylester unter Rühren ca. 16 Stunden auf 70°C bis zur Beendigung der Kohlendioxidentwicklung erwärmt. Das ausgefallene Produkt wird abgesaugt und durch Umlösen aus Aceton und Ausfällen mit Petrolether gereinigt.

Ausbeute: 7 g (30,2% der Theorie), Schmelzpunkt 195—196° C.

Die Verbindungen 1.2 und 1.3 werden analog hergestellt.

Die erfindungsgemäß verwendbaren Stabilisierungsmittel können in wenigstens eine Schicht oder Zwischenschicht eines photographischen Materials eingebracht werden. Sie können beispielsweise den lichtempfindlichen Silberhalogenidemulsionen oder der fertigen Gießlösung zugesetzt werden oder auch zusammen mit der letzten Schutzschicht auf das photographische Material aufgebracht werden. Die dabei verwendete Menge an Stabilisierungsmitteln kann innerhalb weiter Grenzen schwanken. Sie hängt von der Art der Emulsion und dem gewünschten Effekt ab. Im allgemeinen werden mit Mengen von 10 mg bis 5 g, insbesondere von 50 mg bis 500 mg pro Mol Silberhalogenid, die gewünschten Effekte erreicht. Die für jede Emulsion bzw. jedes photographische Material optimale Menge kann in einfacher Weise durch die üblichen Testversuche ermittelt werden.

Die Zugabe der erfindungsgemäß verwendbaren Stabilisierungsmittel zu lichtempfindlichen Silberhalogenidemulsionen kann grundsätzlich zu einem beliebigen Zeitpunkt während der Herstellung bzw. Weiterverarbeitung der Emulsionen erfolgen. In einer bevorzugten Ausführung werden die erfindungsgemäß verwendbaren Stabilisierungsmittel frühestens zur chemischen Reifung der Silberhalogenidemulsion zugesetzt. In einer anderen bevorzugten Ausführungsform werden sie erst nach der chemischen Reifung, vorzugsweise der fertigen Gießlösung zugesetzt.

Die erfindungsgemäß verwendbaren Stabilisierungsmittel können aber auch einem der bei der Verarbeitung photographischer Materialien verwendeten Bäder zugesetzt werden, insbesondere den Bädern, die vor dem Entwicklungsbad eingesetzt werden, bzw. einem Entwicklerbad. Die Stabilisierungsmittel werden in Bädern in Konzentrationen von 25 mg/l bis 1,5 g/l, vorzugsweise von 50 mg/l bis 500 mg/l, eingesetzt.

Photographische Emulsionen bzw. Materialien, die mit den erfindungsgemäßen Stabilisatoren ausgerüstet sind, können darüber hinaus andere Stabilisatoren enthalten, wie z. B. homöopolare oder salzartige Verbindungen des Quecksilbers mit aromatischen oder heterocyclischen Ringen. Als weitere zusätzliche Stabilisatoren sind heterocyclische Mercaptoverbindungen, z. B. Phenylmercaptotetrazol, quaternäre Benzthiazol-Derivate und Benzotriazol geeignet.

In einer bevorzugten Ausführungsform der Erfindung werden die erfindungsgemäß verwendbaren Stabilisierungsmittel in Kombination mit an sich bekannten Stabilisatoren des Indolizin-Typs, vorzugsweise mit Tri- oder Tetraazaindolizinen, und insbesondere mit solchen, die mit wenigstens einer Hydroxyl- und/oder Aminogruppe substituiert sind, eingesetzt. Derartige Indolizine sind beispielsweise in dem Artikel von Birr, Z. Wiss. Phot. 47 (1952), Seiten 2—58 und in der US-A-2 944 902

beschrieben.

Besonders bevorzugte zusätzlich verwendbare Indolizinderivate entsprechen folgender Formel:

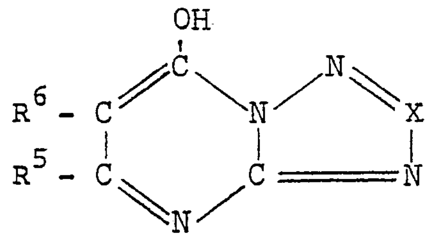

in der bedeuten:

X C—$R^4$ oder N,

$R^4$ und $R^5$ gleich oder verschieden; ein Wasserstoffatom, eine Alkylgruppe, vorzugsweise mit 1 bis 4 C-Atomen, insbesondere Methyl oder eine Cycloalkylgruppe, insbesondere mit nicht mehr als 6 C-Atomen oder eine Aralkylgruppe, insbesondere Benzyl oder eine Arylgruppe, vorzugsweise Phenyl;

$R^6$ ein Wasserstoffatom, eine Alkylgruppe, vorzugsweise mit 1 bis 4 C-Atomen, insbesondere Methyl oder eine Carboxylgruppe oder eine Alkoxycarbonylgruppe.

Die Substituenten $R^4$, $R^5$ und $R^6$ können ihrerseits mit in der Photographie üblichen Substituenten versehen sein; insbesondere kann $R^4$ Hydroxylalkyl sein.

In einer bevorzugten Ausführungsform bedeuten $R^4$ und $R^6$ Wasserstoff und $R^5$ Methyl.

Zusätzliche Stabilisierungsmittel können grundsätzlich den photographischen Materialien bzw. Emulsionen vor, nach oder zu den Zeitpunkten zugesetzt werden, an denen auch die erfindungsgemäßen Stabilisierungsmittel zugesetzt werden.

Besonders bevorzugte zusätzlich verwendbare Stabilisatoren vom Azainden-Typ sind insbesondere:

2.1 4-Hydroxy-6-methyl-1,3,3a,7-tetraazainden,
2.2 4-Hydroxy-5-carboxy-1,3,3a,7-tetraazainden,
2.3 4-Hydroxy-5-carbethoxy-1,3,3a,7-tetraazainden,
2.4 2-$\beta$-Hydroxyethyl-4-hydroxy-6-methyl-1,3,3a,7-tetraazainden,
2.5 2-Methyl-4-hydroxy-1,3,3a,7-tetraazainden,
2.6 4-Hydroxy-6-methyl-1,2,3,3a,7-pentaazainden.

Die erfindungsgemäß verwendbaren Stabilisierungsmittel der Formel I zeichnen sich insbesondere dadurch aus, daß sie nicht nur den Schleier unterdrücken, sondern insbesondere die Gradation stabilisieren. Eine derartige Stabilisierung wird insbesondere bei zusätzlicher Verwendung einer Verbindung gemäß Formel IV erreicht. Falls erforderlich, können der Emulsion zusätzlich weitere zur Steigerung der Gradation geeignete Verbindungen, wie Rhodium- und Cadmium-Verbindungen, zugesetzt werden. Weiterhin vermindern die Verbindungen gemäß Formel I die Empfindlichkeit für Dunkelkammerlicht.

Es wurde weiterhin gefunden, daß Emulsionen mit den erfindungsgemäßen Stabilisierungsmitteln hervorragend zur Herstellung photographischer Materialien durch Auftragen der Emulsion auf einen Träger geeignet sind. Weiterhin eignen sich Emulsionen mit den erfindungsgemäß zu verwendenden Stabilisierungsmitteln für die Herstellung photographischer Bilder durch Auftragen wenigstens einer Emulsionsschicht auf einen photographischen Träger, auf den noch weitere übliche Schichten aufgetragen werden können, bildmäßige Belichtung und Entwicklung.

Die erfindungsgemäß zu verwendenden Stabilisierungsmittel können in den üblichen lichtempfindlichen photographischen Materialien verwendet werden, die für die Herstellung von Schwarz/Weiß-Bildern geeignet sind, z. B. Schwarz/Weiß-Aufnahme- oder Kopiermaterialien, Röntgenmaterialien oder Umkehrmaterialien. Weiterhin können ohne Beeinträchtigung der Stabilisierung Farbkuppler im Material enthalten sein. Die erfindungsgemäß zu verwendenden Stabilisierungsmittel werden vorzugsweise in den sogenannten ausentwickelbaren Materialien verwendet.

Für die vorliegende Erfindung sind die üblichen Silberhalogenidemulsionen geeignet, die aus reinen Silberhalogeniden oder aus Gemischen davon bestehen können. Beispielsweise können die Silberhalogenidkörner aus Silberchlorid, Silberbromid, Silberiodid, Silberchloridbromid, Silberchloridiodid, Silberbromidiodid und Silberchloridbromidiodid bestehen.

Die vorliegende Erfindung ist insbesondere geeignet für kontrastreiche, feinkörnige Emulsionen, besonders für sogenannte Lith-Materialien. Die vorliegende Erfindung ist insbesondere geeignet für Emulsionen für graphische Zwecke, die mindestens 50 Mol-% Silberchlorid, mindestens 5 Mol-% Silberbromid und weniger als 1 Mol-% Silberiodid enthalten. Die erfindungsgemäß stabilisierten Emulsionen sind nicht nur für normale Belichtungen, sondern auch für Kurzzeitbelichtungen (Blitz) und

für Belichtungen mit Laser geeignet. Die Emulsionen können chemisch sensibilisiert werden, z. B. durch Zusatz schwefelhaltiger Verbindungen bei der chemischen Reifung, beispielsweise Allylisothiocyanat, Allylthioharnstoff und Natriumthiosulfat. Als chemische Sensibilisatoren können ferner auch Reduktionsmittel, z. B. die in den belgischen Patentschriften 493 464 und 568 687 beschriebenen Zinnverbindungen, ferner Polyamine wie Diethylentriamin oder Aminoethylsulfinsäure-Derivate, z. B. gemäß der belgischen Patentschrift 547 323 verwendet werden.

Geeignet als chemische Sensibilisatoren sind auch Edelmetalle bzw. Edelmetallverbindungen wie Gold, Platin, Palladium, Iridium, Ruthenium oder Rhodium. Diese Methode der chemischen Sensibilisierung ist in dem Artikel von R. Koslowsky, Z. Wiss. Phot. 46 (1951), Seiten 65–72, beschrieben.

Es ist ferner möglich, die Emulsionen mit Polyalkylenoxid-Derivaten zu sensibilisieren z. B. mit Polyethylenoxid eines Molekulargewichts zwischen 1000 und 20 000, ferner mit Kondensationsprodukten von Alkylenoxiden und aliphatischen Alkoholen, Glykolen, cyclischen Dehydratisierungsprodukten von Hexitolen, mit alkylsubstituierten Phenolen, aliphatischen Carbonsäuren, aliphatischen Aminen, aliphatischen Diaminen und Amiden. Die Kondensationsprodukte haben im allgemeinen ein Molekulargewicht von mindestens 700, vorzugsweise von mehr als 1000. Zur Erzielung besonderer Effekte kann man diese Sensibilisatoren selbstverständlich kombiniert verwenden, wie in der belgischen Patentschrift 537 278 und in der britischen Patentschrift 727 982 beschrieben.

Die Emulsionen können auch optisch sensibilisiert sein, z. B. mit den üblichen Polymethinfarbstoffen wie Neutrocyanine, basischen oder sauren Carbocyaninen, Rhodacyaninen, Hemicyaninen, Styrylfarbstoffen und Oxonolen. Derartige Sensibilisatoren sind in dem Werk von F. M. Hamer, »The Cyanine Dyes and related Compounds«, 1964, Interscience Publishers, John Wiley and Sons, beschrieben. Vorzugsweise werden die Emulsionen für grafische Zwecke mit Merocyaninfarbstoffen sensibilisiert. Geeignete Merocyaninfarbstoffe sind in DE-B-1 234 522 und US-A-2 497 876, 2 519 001, 2 719 152, 3 480 439, 3 752 673, 3 765 900 und 3 765 901 beschrieben.

Die Emulsionen können in der üblichen Weise gehärtet sein, beispielsweise mit Formaldehyd oder halogensubstituierten Aldehyden, die eine Carboxylgruppe enthalten wie Mucobromsäure, Diketonen, Methansulfonsäureester und Dialdehyden. Weiterhin können die photographischen Schichten mit Härtern des Epoxityps, des heterocyclischen Ethylenimins oder des Acryloyltyps gehärtet werden. Beispiele derartiger Härter sind z. B. in der deutschen Offenlegungsschrift 2 263 602 oder in der britischen Patentschrift 1 266 655 beschrieben.

Weiterhin ist es auch möglich, die Schichten gemäß dem Verfahren der deutschen Offenlegungsschrift 2 218 009 zu härten, um photographische Materialien zu erzielen, die für eine Hochtemperaturverarbeitung geeignet sind.

Es ist ferner möglich, die photographischen Schichten bzw. Materialien mit Härtern der Diazin-, Triazin- oder 1,2-Dihydrochinolin-Reihe zu härten, wie in den britischen Patentschriften 1 193 290, 1 251 091, 1 306 544, 1 266 655, der französischen Patentschrift 7 102 716 oder der deutschen Offenlegungsschrift 2 332 317 beschrieben ist. Beispiele derartiger Härter sind alkyl- oder arylsulfonylgruppenhaltige Diazin-Derivate, Derivate von hydrierten Diazinen oder Triazinen wie z. B. 1,3,5-Hexahydrotriazin, fluorsubstituierte Diazin-Derivate wie z. B. Fluorpyrimidin, Ester von 2-substituierten 1,2-Dihydrochinolin- oder 1,2-Dihydroisochinolin-N-carbonsäuren. Brauchbar sind weiterhin Vinylsulfonsäurehärter, Carbodiimid- oder Carbamoylhärter wie z. B. in den deutschen Offenlegungsschriften 2 263 602, 2 225 230 und 1 808 685, der französischen Patentschrift 1 491 807, der deutschen Patentschrift 872 153 und der DD-Patentschrift 7218 beschrieben. Weitere brauchbare Härter sind beispielsweise in der britischen Patentschrift 1 268 550 beschrieben.

Die vorliegende Erfindung kann sowohl für die Herstellung schwarzweißer als auch farbiger photographischer Bilder angewendet werden. Farbige photographische Bilder können z. B. nach dem bekannten Prinzip der chromogenen Entwicklung in Anwesenheit von Farbkupplern, die mit dem Oxidationsprodukt von farbgebenden p-Phenylendiamin-Entwicklern unter Bildung von Farbstoffen reagieren, hergestellt werden.

Die vorliegende Erfindung ist auch geeignet für die Stabilisierung eines Aufzeichnungsmaterials mit variabler Gradation, wie es z. B. aus der DE-C-1 597 476 bekannt ist.

Die Emulsionen können auf die üblichen Schichtträger aufgetragen werden, z. B. auf Träger aus Celluloseestern wie Celluloseacetat oder Celluloseacetobutyrat, ferner Polyester, insbesondere Polyethylenterephthalat oder Polycarbonate, insbesondere auf Basis von Bis-phenylolpropan. Geeignet sind ferner Papierträger, die gegebenenfalls wasserundurchlässige Polyolefinschichten, z. B. aus Polyethylen oder Polypropylen, enthalten können, ferner Träger aus Glas oder Metall.

Für eine Schwarz/Weiß-Entwicklung sind die üblichen bekannten Schwarz/Weiß-Entwicklerverbindungen geeignet, wie z. B. Hydrochinone, 3-Pyrazolidone, Aminophenole und Brenzkatechine.

### Beispiel 1

Nach dem Doppeleinlaufverfahren wurde eine Silberhalogenidemulsion mit 83,6 Mol-% Chlorid, 16 Mol-% Bromid und 0,4 Mol-% Iodid hergestellt, die 0,06 ppm $Rh^{+++}$-Ionen, bezogen auf den

Silbergehalt, enthält. Die Silberhalogenidkörner wiesen eine enge Kornverteilung auf und die mittlere Korngröße betrug 0,27 µ. Die Emulsion wurde in bekannter Weise mit Gold- und Schwefelverbindungen gereift und danach mit einem Benzoxazolinyliden-thiohydantoin-dimethinmerocyaninfarbstoff sensibilisiert. Weiterhin wurde die Emulsion mit einem Netzmittel und einem Härtungsmittel versehen. Der Emulsion wurden die aus der folgenden Tabelle II ersichtlichen Mengen an Stabilisierungsmittel einschließlich Cadmiumchlorid zugesetzt.

Die erhaltenen Emulsionen wurden auf einen Polyethylenterephthalatträger mit einem Silberauftrag (berechnet als Silbernitrat) von 7 g/m² vergossen. Der Gelatineauftrag betrug 3,5 g/m². Die erhaltenen Materialien wurden frisch und nach 5tägiger Lagerung bei 57°C und 34% rel. Luftfeuchtigkeit in üblicher Weise in einem Mark-VI-Sensitometer der Firma EG & G, Inc., Boston, Mass./USA, $10^{-4}$ Sekungen lang belichtet und in einem Entwickler I der folgenden Zusammensetzung bei 35°C 35 Sekunden lang entwickelt. Anschließend wurde in üblicher Weise fixiert und gewässert.

Entwickler I (g pro Liter)

| | |
|---|---|
| $K_2SO_3$ | 36,0 g |
| 1-Phenyl-3-pyrazolidon | 0,3 g |
| Hydrochinon | 10,0 g |
| Pottasche | 20,0 g |
| KBr | 2,6 g |
| 1-Phenyl-5-mercaptotetrazol | 15,0 mg |

Es wurden die aus Tabelle II ersichtlichen Ergebnisse erhalten.

Tabelle II

| Stabilisierungsmittel/g/mol Silberhalogenid | Frischprüfung | | | | Lagerung 5 Tage bei 57°C und 34% rel. Luftfeuchtigkeit | | | |
|---|---|---|---|---|---|---|---|---|
| | S | E | $\gamma_v$ | $\gamma$ | S | E | $\gamma_v$ | $\gamma$ |
| 0,075 g Verbindung 2.1<br>7 g $CdCl_2 \cdot H_2O$ | 0,11 | 100 | 1,37 | 5,90 | 0,21 | 140 | 1,40 | 5,10 |
| 0,075 g Verbindung 2.1<br>7 g $CdCl_2 \cdot H_2O$<br>0,150 g Verbindung 1.1 | 0,08 | 100 | 1,60 | 5,70 | 0,11 | 160 | 1,67 | 5,00 |

E = relative Empfindlichkeit bei der Dichte 2,0 über Schleier; eine Verdopplung der angegebenen Werte entspricht einer Verdopplung der Empfindlichkeit.
$\gamma_v$ = Gradation zwischen der Dichte 0,1 über Schleier und der Dichte 0,5 über Schleier.
$\gamma$ = Gradation zwischen der Dichte 1,0 über Schleier und der Dichte 2,5 über Schleier.
S = Schleier.

Verbindung 1.1 stabilisiert nicht nur den Schleier, sondern vor allem auch die steile Gradation, insbesondere $\gamma_v$.

## Beispiel 2

Eine Silberhalogenidemulsion der in Beispiel 1 beschriebenen Zusammensetzung wurde wie in Beispiel 1 angegeben hergestellt und weiterverarbeitet mit der Abänderung, daß die Emulsionen nicht auf einen Polyethylenterephthalatträger, sondern auf eine beidseitig mit Polyethylen beschichtete Papierunterlage vergossen wurden. Der Silberauftrag (als Silbernitrat) betrug 3 g/m², der Gelatineauftrag 1,7 g/m². Nach der in Beispiel 1 beschriebenen Verarbeitung wurden die aus der folgenden Tabelle III ersichtlichen Werte erhalten.

Tabelle III

| Stabilisierungsmittel/g/mol Silberhalogenid | Frischprüfung | | | | Lagerung 5 Tage bei 57°C und 34 % rel. Luftfeuchtigkeit | | | |
|---|---|---|---|---|---|---|---|---|
| | S | E | $\gamma_v$ | $\gamma$ | S | E | $\gamma_v$ | $\gamma$ |
| 0,075 g Verbindung 2.1<br>7 g $CdCl_2 \cdot H_2O$ | 0,18 | 100 | 1,77 | 2,35 | 0,42 | 50 | 1,24 | 1,30 |
| 0,075 g Verbindung 2.1<br>7 g $CdCl_2 \cdot H_2O$<br>0,150 g Verbindung 1.1 | 0,13 | 70 | 1,34 | 2,20 | 0,26 | 100 | 1,39 | 1,90 |

E = relative Empfindlichkeit bei Dichte 0,8 über Schleier.
$\gamma_v$ = Gradation zwischen der Dichte 0,1 über Schleier und der Dichte 0,3 über Schleier.
$\gamma$ = Gradation zwischen der Dichte 0,3 über Schleier und der Dichte 1,1 über Schleier.
S = Schleier.

Verbindung 1.1 stabilisiert nicht nur den Schleier, sondern vor allem auch die steile Gradation, insbesondere $\gamma_v$.

## Beispiel 3

Nach dem in Beispiel 1 angegebenen Verfahren wurde eine Silberhalogenidemulsion mit der einzigen Abänderung hergestellt, daß sie 0,10 ppm $Rh^{+++}$-Ionen, bezogen auf den Silbergehalt, enthält. Dabei wurden den einzelnen Anteilen der Emulsion die folgenden Mengen an Stabilisierungsmitteln pro Mol Silberhalogenid zugegeben:

Probe 1: 7 g $CdCl_2 \cdot H_2O$ + 75 mg Verbindung 2.1
Probe 2: 7 g $CdCl_2 \cdot H_2O$ + 170 mg Verbindung 1.1 + 75 mg Verbindung 2.1
Probe 3: 7 g $CdCl_2 \cdot H_2O$ + 255 mg Verbindung 1.1 + 75 mg Verbindung 2.1

Die erhaltenen Emulsionen wurden auf einen Polyethylenterephthalatträger aufgetragen. Die erhaltenen Materialien wurden wie in Beispiel 1 angegeben belichtet und in dem in Beispiel 1 angegebenen Entwickler I unter den aus Tabelle IV ersichtlichen Bedingungen weiterverarbeitet. Dabei wurden die Materialien entweder frisch belichtet und verarbeitet oder nach 36stündiger Lagerung bei 57°C und 34% rel. Feuchtigkeit bzw. nach 5tätiger Lagerung bei 57°C und 34% rel. Feuchtigkeit.

Tabelle IV

| | Entwicklung: 35 Sekunden bei 35°C | | | Entwicklung: 60 Sekunden bei 35°C | | |
|---|---|---|---|---|---|---|
| | Probe 1 | 2 | 3 | Probe 1 | 2 | 3 |
| Frischprüfung | | | | | | |
| S | 0,11 | 0,08 | 0,07 | 0,14 | 0,09 | 0,09 |
| $\gamma$ | 5,90 | 5,70 | 5,35 | 5,50 | 5,75 | 5,30 |
| $\gamma_v$ | 1,57 | 1,60 | 1,61 | 1,35 | 1,40 | 1,47 |
| E | 100 | 81 | 102 | 79 | 107 | 91 |
| Lagerung 36 Stunden: | | | | | | |
| S | 0,17 | 0,09 | 0,09 | 0,21 | 0,11 | 0,12 |
| $\gamma$ | 5,35 | 4,90 | 5,00 | 4,50 | 5,25 | 5,95 |
| $\gamma_v$ | 1,44 | 1,32 | 1,34 | 1,20 | 1,34 | 1,40 |
| E | 110 | 138 | 155 | 148 | 174 | 166 |
| Lagerung 5 Tage | | | | | | |
| S | 0,21 | 0,10 | 0,11 | 0,27 | 0,15 | 0,12 |
| $\gamma$ | 5,10 | 4,95 | 5,00 | 5,20 | 5,15 | 5,25 |
| $\gamma_v$ | 1,40 | 1,34 | 1,67 | 1,17 | 1,45 | 1,32 |
| E | 135 | 138 | 162 | 158 | 182 | 162 |

Die in Tabelle IV verwendeten Symbole S, $\gamma$, $\gamma_v$ und E haben die in Beispiel 1 angegebene Bedeutung.

Beispiel 4

Beispiel 3 wurde wiederholt mit der Abänderung, daß nunmehr den einzelnen Anteilen der Emulsion die folgenden Mengen an Stabilisierungsmitteln pro Mol Silberhalogenid zugegeben wurden:

Probe

1 7 g $CdCl_2 \cdot H_2O$ + 17 mg 1-Phenyl-5-mercaptotetrazol
2 wie Probe 1 + 85 mg Verbindung 1.1
3 wie Probe 1 + 170 mg Verbindung 1.1
4 wie Probe 1 + 255 mg Verbindung 1.1

Zuzüglich enthält jede Probe pro Mol Silberhalogenid 75 mg Verbindung 2.1.

Die erhaltenen Emulsionen wurden auf ein beidseits mit Polyethylen beschichtetes Papier aufgetragen. Der Silberauftrag (berechnet als Silbernitrat) betrug 3 g $AgNO_3/m^2$. Die erhaltenen Materialien wurden wie in Beispiel 1 angegeben belichtet und in dem in Beispiel 1 angegebenen Entwickler I unter den aus Tabelle V ersichtlichen Bedingungen weiterverarbeitet. Dabei wurden die Materialien entweder frisch belichtet und verarbeitet oder nach 36stündiger Lagerung bei 57° C und 34% rel. Feuchtigkeit bzw. nach 5tägiger Lagerung bei 57° C und 34% rel. Feuchtigkeit. Die in Tabelle V verwendeten Symbole haben die in Beispiel 2 angegebene Bedeutung; $D_{max}$ bedeutet die erreichte maximale Dichte.

Tabelle V

| | Entwicklung: 35 Sekunden bei 35°C | | | | Entwicklung: 60 Sekunden bei 35°C | | | |
|---|---|---|---|---|---|---|---|---|
| | Probe 1 | 2 | 3 | 4 | Probe 1 | 2 | 3 | 4 |
| Frischprüfung | | | | | | | | |
| S | 0,18 | 0,18 | 0,13 | 0,12 | 0,24 | 0,22 | 0,19 | 0,18 |
| $\gamma$ | 2,35 | 1,86 | 2,20 | 2,30 | 1,85 | 1,79 | 2,15 | 1,94 |
| $\gamma_v$ | 1,77 | 1,38 | 1,34 | 1,18 | 1,14 | 1,25 | 1,55 | 1,32 |
| E | 100 | 70 | 79 | 72 | 64 | 75 | 75 | 57 |
| $D_{max}$ | 1,36 | 1,36 | 1,40 | 1,38 | 1,34 | 1,38 | 1,40 | 1,38 |
| Lagerung 36 Stunden | | | | | | | | |
| S | 0,30 | 0,23 | 0,18 | 0,18 | 0,42 | 0,32 | 0,26 | 0,24 |
| $\gamma$ | 1,75 | 1,77 | 2,34 | 2,00 | 1,58 | 1,92 | 2,26 | 1,82 |
| $\gamma_v$ | 1,16 | 1,18 | 1,28 | 1,18 | 1,13 | 1,30 | 1,34 | 1,44 |
| E | 75 | 112 | 105 | 91 | 95 | 120 | 120 | 145 |
| $D_{max}$ | 1,58 | 1,40 | 1,40 | 1,40 | 1,58 | 1,38 | 1,36 | 1,38 |
| Lagerung 5 Tage | | | | | | | | |
| S | 0,42 | 0,36 | 0,26 | 0,32 | 0,54 | 0,46 | 0,40 | 0,40 |
| $\gamma$ | 1,30 | 1,82 | 1,90 | 1,82 | 1,34 | 1,80 | 1,77 | 1,88 |
| $\gamma_v$ | 1,24 | 1,36 | 1,39 | 1,39 | 1,34 | 1,37 | 1,45 | 1,27 |
| E | 70 | 100 | 98 | 105 | – | 89 | 95 | 89 |
| $D_{max}$ | 1,38 | 1,38 | 1,42 | 1,42 | 1,36 | 1,38 | 1,88 | 1,40 |

## Beispiel 5

Eine Silberhalogenidemulsion der in Beispiel 1 beschriebenen Zusammensetzung wird wie in Beispiel 1 angegeben hergestellt und weiterverarbeitet mit dem Unterschied, daß den einzelnen Anteilen der Emulsion pro Mol Silberhalogenid die in der Tabelle VI angegebenen Mengen an Stabilisierungsmittel zugegeben wurden.

Die erhaltenen Materialien wurden entweder frisch belichtet und verarbeitet oder nach 5tägiger Lagerung bei 57°C und 34% rel. Feuchtigkeit belichtet und verarbeitet wie in Beispiel 1 beschrieben. Die relative Empfindlichkeit für Dunkelkammerlicht wurde nach entsprechender Belichtung und Verarbeitung festgestellt mit dem Unterschied, daß die Belichtung durch ein rotes Dunkelkammerfilter erfolgte.

Es wurden die aus Tabelle VI ersichtlichen Ergebnisse erhalten. Die in Tabelle VI verwendeten Symbole S, $\gamma$, $\gamma_v$ und E haben die in Beispiel 1 angegebene Bedeutung.

Tabelle VI

| Stabilisierungsmittel g/Mol Silberhalogenid | Frischprüfung | | | | | Lagerung 5 Tage 57°C/34 % rel. Feuchtigkeit | | | | |
|---|---|---|---|---|---|---|---|---|---|---|
| | S | E | $\gamma_v$ | $\gamma$ | Dk.E*) | S | E rel. | $\gamma_v$ | $\gamma$ | Dk.E*) |
| 7 g $CdCl_2H_2O$ | 0.02 | 100 | 1.95 | 7.30 | 100 | 0.06 | 100 | 1.65 | 8.30 | 220 |
| 1,1 g Verbindung 2.1 | 0.02 | 135 | 1.50 | 6.60 | 240 | 0.06 | 110 | 1.50 | 7.00 | 270 |
| 1,1 g Verbindung 2.1 + 170 mg Verbindung 1.1 | 0.02 | 105 | 2.00 | 6.30 | 160 | 0.08 | 110 | 1.70 | 6.80 | 190 |
| 1,1 g Verbindung 2.1 + 255 mg Verbindung 1.1 | 0.02 | 106 | 2.05 | 6.35 | 110 | 0.04 | 105 | 1.70 | 7.10 | 160 |

*) Dk.E = relative Dunkelkammerempfindlichkeit.

Die Ergebnisse zeigen, daß die erfindungsgemäß zu verwendenden Stabilisierungsmittel die Gradation in den Spitzlichtern erhöhen und daß eine verminderte Empfindlichkeit für Dunkelkammerlicht erhalten wird. Weiterhin wird in Kombination mit einem Tetraazainden-Stabilisator ein besseres Ergebnis als in Kombination mit Cadmiumchlorid erhalten.

Beispiel 6

Zu mehreren aliquoten Anteilen einer ammoniakalischen Silberbromidjodidemulsion (2 Mol-% Jodid), die pro kg 180 g Silbernitrat enthält, werden 3,6 mmol der Verbindung 2.1 bzw. 3.6 mmol der Verbindung 1.1 pro kg Emulsion zugegeben. Die so erhaltenen Emulsionen werden auf einen üblichen Filmträger aufgetragen und getrocknet.

Die Materialien werden wie in Beispiel 1 angegeben gelagert, hinter einem Stufenkeil belichtet und in dem im folgenden angegebenen Entwickler bei 35° C 90 Sekunden lang entwickelt. Der verwendete Entwickler wurde hergestellt durch Mischen von 1000 ml der im folgenden angegebenen Lösung A mit 2800 ml Wasser, 100 ml der Lösung B und 100 ml der Lösung C (pH-Wert der Mischung: 10,35).

Lösung A

| | |
|---|---|
| Pottasche (40%ige Lösung) | 165 ml |
| Kaliumsulfit (65%ige Lösung) | 346 ml |
| Hydrochinon | 112 g |
| 1-Phenyl-5-mercaptotetrazol | 40 mg |
| Kaliumcarbonat (sicc.) | 60 g |
| KCl | 3,4 g |
| Diethylenglykol | 10 ml |
| mit Wasser auf 1000 ml | |

Lösung B

| | |
|---|---|
| Eisessig | 45 ml |
| 1-Phenyl-pyrazolidin-3-on | |
| mit Ethylenglykol auf 100 ml | 6,2 g |

Lösung C

| | |
|---|---|
| Glutardialdehyd (25%ige Lösung) | 80 ml |
| Kaliummetabisulfit | |
| mit Wasser auf 100 ml | 36 g |

Es werden die aus Tabelle VII ersichtlichen Ergebnisse erhalten.

Tabelle VIII

| Stabilisierungsmittel/ kg Emulsion | Frischprüfung | | | Gelagertes Material | | |
|---|---|---|---|---|---|---|
| | S | E | γ | S | E | γ |
| Ohne Zusatz | 0,21 | 100 | 3.11 | 2,75 | –*) | –*) |
| 540 mg Verbindung 2.1 | 0,20 | 87 | 3.65 | 0,39 | 102 | 2.94 |
| 702 mg Verbindung 1.1 | 0,19 | 83 | 3.55 | 0,23 | 95 | 3.34 |

E = relative Empfindlichkeit bei der Dichte 0,1 über Schleier.
S = Schleier.
*) infolge der hohen Schleier nicht meßbar.

**Patentansprüche**

1. Lichtempfindliche photographische Silberhalogenidemulsion, gekennzeichnet durch den Gehalt an einer Verbindung der folgenden Formel I

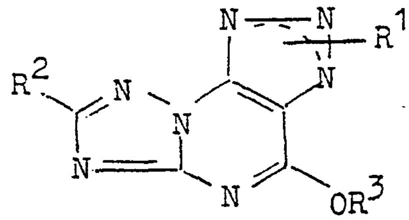

I

worin bedeuten:

$R^1$ H; Alkyl; Cycloalkyl; Aryl, Aralkyl; Carbamoyl; Alkoxycarbonyl;
$R^2$ H; Alkyl; Aralkyl; Cycloalkyl; Aryl;
$R^3$ ein Kation.

2. Emulsion nach Anspruch 1, worin bedeuten:

$R^1$ Wasserstoff; Alkyl mit 1 bis 4 C-Atomen; Cycloalkyl mit maximal 6 C-Atomen; Phenyl; Benzyl; Carbamoyl; Alkoxycarbonyl mit maximal 6 C-Atomen;
$R^2$ Wasserstoff, Alkyl mit max. 6 C-Atomen; Benzyl, Cycloalkyl mit max. 6 C-Atomen; Phenyl;
$R^3$ Wasserstoff, $NH_4^+$ oder ein Metallkation.

3. Emulsion nach Anspruch 1, worin bedeuten:

$R^1$ H; Alkoxycarbonyl; Carbamoyl;
$R^2$ H; Methyl; Phenyl;
$R^3$ N; Natrium; Kalium; $NH_4$.

4. Verfahren zur Herstellung einer Silberhalogenidemulsion durch Fällen des Silberhalogenids in Gegenwart eines Schutzkolloids, gegebenenfalls physikalische Reifung, Koagulierung, Redispergierung und chemische Reifung, dadurch gekennzeichnet, daß frühestens zur chemischen Reifung eine Verbindung der folgenden Formel I zugesetzt wird:

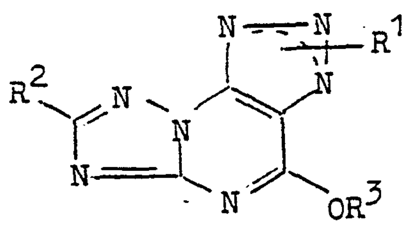

I

worin bedeuten:

$R^1$ H, Alkyl, Cycloalkyl, Aryl, Aralkyl, Carbamoyl, Alkoxycarbonyl;
$R^2$ H, Alkyl, Aralkyl, Cycloalkyl; Aryl;
$R^3$ ein Kation.

5. Photographisches Material bestehend aus einem Schichtträger und wenigstens einer darauf aufgetragenen lichtempfindlichen Silberhalogenidemulsionsschicht und gegebenenfalls weiteren Schichten, dadurch gekennzeichnet, daß wenigstens eine Schicht eine Verbindung der folgenden Formel I enthält:

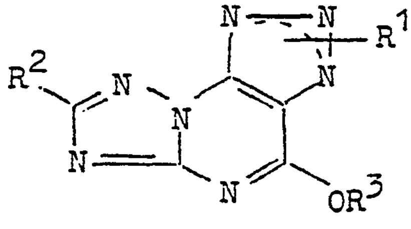

I

worin bedeuten:

$R^1$ H, Alkyl, Cycloalkyl, Aryl, Aralkyl, Carbamoyl, Alkoxycarbonyl;
$R^2$ H, Alkyl, Aralkyl, Cycloalkyl; Aryl;
$R^3$ ein Kation.

6. Material nach Anspruch 5, worin bedeuten:

$R^1$ Wasserstoff; Alkyl mit 1 bis 4 C-Atomen; Cycloalkyl mit maximal 6 C-Atomen; Phenyl; Benzyl; Carbamoyl; Alkoxycarbonyl mit maximal 6 C-Atomen;
$R^2$ Wasserstoff, Alkyl mit max. 6 C-Atomen; Benzyl; Cycloalkyl mit max. 6 C-Atomen; Phenyl;
$R^3$ Wasserstoff, $NH_4^+$ oder ein Metallkation.

7. Material nach Anspruch 5, worin bedeuten:

$R^1$ H; Alkoxycarbonyl; Carbamoyl;
$R^2$ H; Methyl; Phenyl;
$R^3$ H; Natrium; Kalium; $NH_4$.

8. Material nach Anspruch 5, worin bedeuten:

$R^1$ und $R^2$ Wasserstoff;
$R^3$ Na.

9. Material nach Anspruch 5, dadurch gekennzeichnet, daß zusätzlich eine Verbindung der folgenden Formel IV enthalten ist

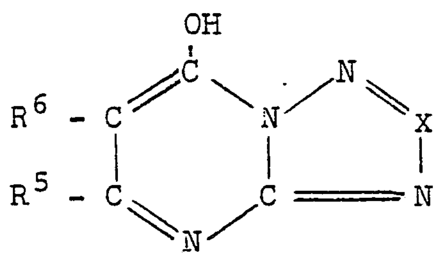

IV

worin bedeuten:

X C—$R^4$ oder N;
$R^4$ und $R^5$ ein Wasserstoffatom, eine Alkylgruppe, eine Cycloalkylgruppe, eine Aralkylgruppe oder eine Arylgruppe;
$R^6$ ein Wasserstoffatom, eine Alkylgruppe, eine Carboxygruppe oder eine Alkoxycarbonylgruppe.

10. Verfahren zur Herstellung photographischer Bilder durch bildmäßige Belichtung und Entwicklung eines photographischen Materials, dadurch gekennzeichnet, daß die Entwicklung durchgeführt wird in Gegenwart einer Verbindung der folgenden Formel I:

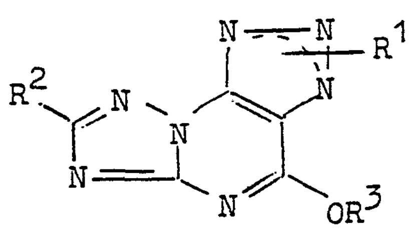

I

worin bedeuten:

$R^1$ H, Alkyl, Cycloalkyl, Aryl, Aralkyl, Carbamoyl, Alkoxycarbonyl;
$R^2$ H, Alkyl, Aralkyl, Cycloalkyl; Aryl;
$R^3$ ein Kation.

**Claims**

1. Light-sensitive photographic silver halide emulsion, characterised in that it contains a compound of the following formula I

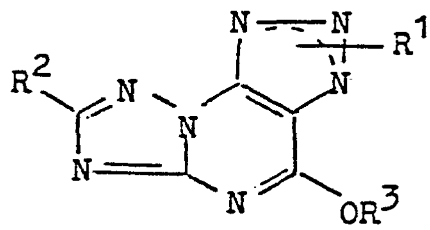

I

wherein

$R^1$ denotes H; alkyl; cycloalkyl; aryl, aralkyl; carbamoyl; alkoxycarbonyl;
$R^2$ denotes H; alkyl; aralkyl; cycloalkyl; aryl;
$R^3$ denotes a cation.

2. Emulsion according to Claim 1, wherein:

$R^1$ denotes hydrogen; alkyl with 1 to 4 C atoms; cycloalkyl with not more than 6 C atoms; phenyl; benzyl; carbamoyl; alkoxycarbonyl with not more than 6 C atoms;
$R^2$ denotes hydrogen, alkyl with not more than 6 C atoms; benzyl, cycloalkyl with not more than 6 C atoms; phenyl;
$R^3$ denotes hydrogen, $NH_4^+$ or a metal cation.

3. Emulsion according to Claim 1, wherein

$R^1$ denotes H; alkoxycarbonyl; carbamoyl;
$R^2$ denotes H; methyl; phenyl;
$R^3$ denotes H; sodium; potassium; $NH_4$.

4. Process for the preparation of a silver halide emulsion by precipitation of the silver halide in the presence of a protective colloid, optionally physical ripening, coagulation, redispersion and chemical ripening, characterised in that a compound of the following formula I

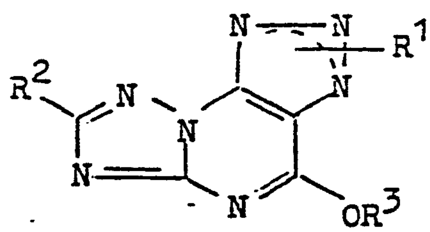

I

wherein

$R^1$ denotes H, alkyl, cycloalkyl, aryl, aralkyl, carbamoyl, alkoxycarbonyl;
$R^2$ denotes H, alkyl, aralkyl, cycloalkyl; aryl;
$R^3$ denotes a cation,

is added at the earliest at the stage of chemical ripening.

5. Photographic material consisting of a support layer and at least one light-sensitive silver halide emulsion layer applied to it and optionally additional layers, characterised in that at least one layer contains a compound of the following formula I wherein

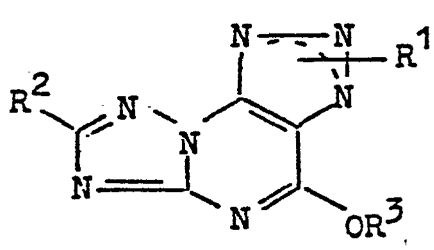

I

$R^1$ denotes H, alkyl, cycloalkyl, aryl, aralkyl, carbamoyl, alkoxycarbonyl;
$R^2$ denotes H, alkyl, aralkyl, cycloalkyl; aryl;
$R^3$ denotes a cation.

6. Material according to Claim 5, wherein

$R^1$ denotes hydrogen; alkyl with 1 to 4 C atoms; cycloalkyl with not more than 6 C atoms; phenyl; benzyl; carbamoyl; alkoxycarbonyl with not more than 6 C atoms;
$R^2$ denotes hydrogen, alkyl with not more than 6 C atoms; benzyl; cycloalkyl with not more than 6 C atoms; phenyl;
$R^3$ denotes hydrogen, $NH_4^+$ or a metal cation.

7. Material according to Claim 5, wherein:

$R^1$ denotes H; alkoxycarbonyl; carbamoyl;
$R^2$ denotes H; methyl; phenyl;
$R^3$ denotes H; sodium; potassium; $NH_4$.

8. Material according to Claim 5, wherein:

$R^1$ and $R^2$ denote hydrogen;
$R^3$ denotes Na.

9. Material according to Claim 5, characterised in that the emulsion additionally contains a compound of the following formula IV

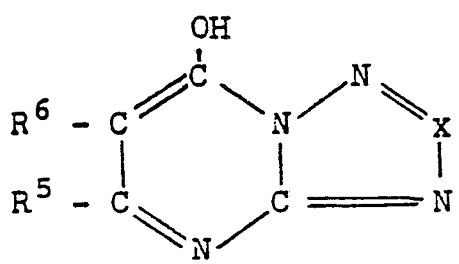

IV

wherein:

X denotes $C-R^4$ or N;
$R^4$ and $R^5$ denote a hydrogen atom, an alkyl group, a cycloalkyl group, an aralkyl group or an aryl group;
$R^6$ denotes a hydrogen atom, an alkyl group, a carboxy group or an alkoxycarbonyl group.

10. Process for the preparation of photographic images by imagewise exposure and development of a photographic material, characterised in that development is carried out in the presence of a compound of the following formula I:

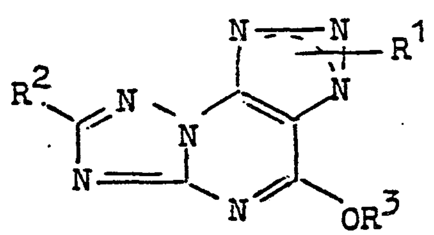

I

wherein

$R^1$ denotes H, alkyl, cycloalkyl, aryl, aralkyl, carbamoyl, alkoxycarbonyl;
$R^2$ denotes H, alkyl, aralkyl, cycloalkyl; aryl;
$R^3$ denotes a cation.

**Revendications**

1. Emulsion photographique photosensible à l'halogénure d'argent, caractérisée en ce qu'elle contient un composé répondant à la formule I ci-après:

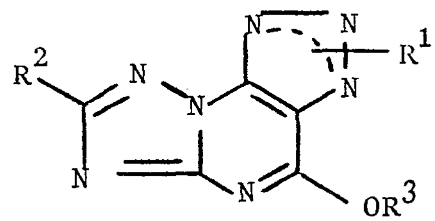

dans laquelle:

$R^1$ représente H; un groupe alkyle; un groupe cycloalkyle; un groupe aryle; un groupe aralkyle; un groupe carbamoyle ou un groupe alcoxycarbonyle;
$R^2$ représente H; un groupe alkyle; un groupe aralkyle; un groupe cycloalkyle ou un groupe aryle;
$R^3$ représente un cation.

2. Emulsion suivant la revendication 1, dans laquelle:

$R^1$ représente un atome d'hydrogène; un groupe alkyle contenant 1 à 4 atomes de carbone; un groupe cycloalkyle contenant 6 atomes de carbone maximum; un groupe phényle; un groupe benzyle; un groupe carbamoyle; un groupe alcoxycarbonyle contenant 6 atomes de carbone maximum;
$R^2$ représente un atome d'hydrogène; un groupe alkyle contenant 6 atomes de carbone maximum; un groupe benzyle; un groupe cycloalkyle contenant 6 atomes de carbone maximum ou un groupe phényle;
$R^3$ représente un atome d'hydrogène, $NH_4^+$ ou un cation d'un métal.

3. Emulsion suivant la revendication 1, dans laquelle:

$R^1$ représente H; un groupe alcoxycarbonyle ou un groupe carbamoyle;
$R^2$ représente H; un groupe méthyle ou un groupe phényle;
$R^3$ représente H; le sodium; le potassium ou $NH_4$.

4. Procédé de préparation d'une émulsion à l'halogénure d'argent par précipitation de l'halogénure d'argent en présence d'un colloïde protecteur et éventuellement par maturation physique, coagulation, redispersion et maturation chimique, caractérisé en ce que, au plus tôt lors de la maturation chimique, on ajoute un composé répondant à la formule I ci-après:

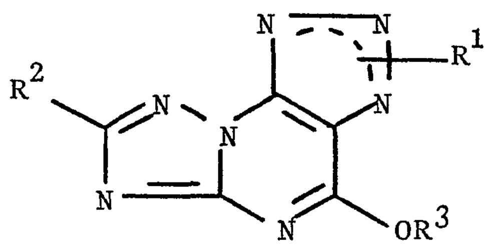

dans laquelle:

$R^1$ représente H, un groupe alkyle, un groupe cycloalkyle, un groupe aryle, un groupe aralkyle, un groupe carbamoyle ou un groupe alcoxycarbonyle;
$R^2$ représente H, un groupe alkyle, un groupe aralkyle, un groupe cycloalkyle ou un groupe aryle;
$R^3$ représente un cation.

5. Elément photographique comprenant un support de couches et au moins une couche photosensible d'émulsion à l'halogénure d'argent déposée sur ce support et éventuellement d'autres couches, caractérisé en ce qu'au moins une couche contient un composé répondant à la formule I ci-après:

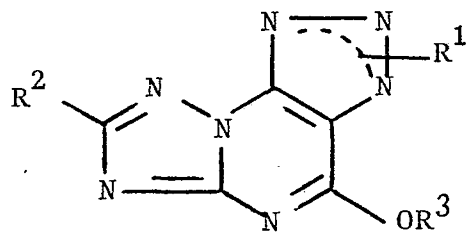

dans laquelle:

$R^1$ représente H, un groupe alkyle, un groupe cycloalkyle, un groupe aryle, un groupe aralkyle, un groupe carbamoyle ou un groupe alcoxycarbonyle;
$R^2$ représente H, un groupe alkyle, un groupe aralkyle, un groupe cycloalkyle ou un groupe aryle;
$R^3$ représente un cation.

6. Elément suivant la revendication 5, dans lequel:

$R^1$ représente un atome d'hydrogène; un groupe alkyle contenant 1 à 4 atomes de carbone; un groupe cycloalkyle contenant 6 atomes de carbone maximum; un groupe phényle; un groupe benzyle; un groupe carbamoyle; un groupe alcoxycarbonyle contenant 6 atomes de carbone maximum;
$R^2$ représente un atome d'hydrogène, un groupe alkyle contenant 6 atomes de carbone maximum; un groupe benzyle; un groupe cycloalkyle contenant 6 atomes de carbone maximum; ou un groupe phényle;
$R^3$ représente un atome d'hydrogène, $NH_4^+$ ou un cation d'un métal.

7. Elément suivant la revendication 5, dans lequel:

$R^1$ représente H; un groupe alcoxycarbonyle ou un groupe carbamoyle;
$R^2$ représente H; un groupe méthyle ou un groupe phényle;
$R^3$ représente H; le sodium; le potassium ou $NH_4$.

8. Elément suivant la revendication 5, dans lequel:

$R^1$ et $R^2$ représentent chacun l'hydrogène;
$R^3$ représente Na.

9. Elément suivant la revendication 5, caractérisé en ce qu'il contient, en outre, un composé répondant à la formule IV ci-après:

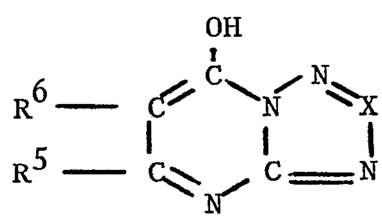

dans laquelle:

X représente C—$R^4$ ou N;
$R^4$ et $R^5$ représentent un atome d'hydrogène, un groupe alkyle, un groupe cycloalkyle, un groupe aralkyle ou un groupe aryle;
$R^6$ représente un atome d'hydrogène, un groupe alkyle, un groupe carboxy ou un groupe alcoxycarbonyle.

10. Procédé de formation d'images photographiques par exposition sous forme d'une image et développement d'un élément photographique, caractérisé en ce qu'on effectue le développement en présence d'un composé répondant à la formule I ci-après:

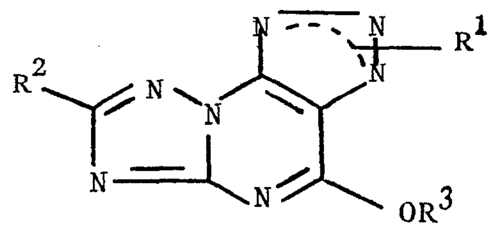

dans laquelle:

$R^1$ représente H, un groupe alkyle, un groupe cycloalkyle, un groupe aryle, un groupe aralkyle, un groupe carbamoyle ou un groupe alcoxycarbonyle;
$R^2$ représente H, un groupe alkyle, un groupe aralkyle, un groupe cycloalkyle ou un groupe aryle;
$R^3$ représente un cation.